# EUROPEAN PATENT SPECIFICATION

(11) **EP 2 767 918 B1**
(45) Date of publication and mention of the grant of the patent: **13.11.2019**
(21) Application number: 14155347.9
(22) Date of filing: 17.02.2014
(51) Int. Cl.: A61G 7/00, H04B 5/00

(54) **Direct patient association**
Direkte Patientenzuordnung
Association de patient directe

(30) Priority: 19.02.2013 US 201361766212 P
(43) Date of publication of application: 20.08.2014
(73) Proprietor: Hill-Rom Services, Inc., Batesville, IN 47006-9167 (US)
(72) Inventor: Kim, Peter S., West Chester, OH 45069 (US); Agdeppa, Eric D., Cincinnati, OH 45249 (US); Ribble, David Lance, Indianapolis, IN 46202 (US); Tallent, Dan R., Hope, IN 47246 (US); Schuman, Richard Joseph, Cary, NC 27511 (US); Huster, Keith A., Sunman, IN 47041 (US); Bishop, William B., Apex, NC 27539 (US)
(74) Representative: Findlay, Alice Rosemary

(56) References cited:
- WO-A1-2011/100760
- WO-A2-2006/035351
- US-A1- 2007 120 689
- US-A1- 2008 120 784
- US-A1- 2010 174 229
- US-A1- 2012 089 419

## Description

The present disclosure relates to a system and method for associating a patient with medical equipment and/or a location in a healthcare facility. More particularly, the present disclosure relates to associating a patient with a room or with a hospital bed that is located in a room of a healthcare facility.

In current healthcare communication systems, one or more of patient-to-location, patient-to-equipment, and patient-to-bed associations are oftentimes entered manually be caregivers in multiple computer devices of different portions of the overall healthcare communications system. For example, patient information (ID) or data is entered into a computer of an admission/discharge/transfer (ADT) system when a patient is admitted into a healthcare facility. The patient room location is typically assigned to the patient at this time and data regarding the room assignment may be entered into the ADT computer.

Nurse call systems that include a communication link to a number of hospital beds in a unit or ward and that receive bed status data are known. Patient ID is sometimes entered into a computer of the nurse call system manually and is associated manually with a particular hospital bed that is at the location to which the patient has been assigned. In some prior art systems, the nurse call system computer operates to determine a bed-to-location association based on a bed ID transmitted from the bed via a unit or module mounted in the room. The unit transmits both the bed ID and unit ID and the nurse call system computer correlates or associates these ID's with the room number or location.

Healthcare facilities also typically include an Electronic Medical Records (EMR) system. The EMR systems in such facilities typically associate a medical record number (MRN) with each patient and that association may be manually entered either at the ADT computer or at an EMR computer or both. The association between various pieces of medical equipment, including hospital beds, and the assigned patient may also be entered manually at an EMR computer in some healthcare communication systems.

In some systems, one computer may receive patient-to-location and/or patient-to-equipment association information from another computer and then, a caregiver may manually verify the association at the receiving computer. It has also been contemplated that confirmation of a patient-to-bed or patient-to-room association may be done manually at a hospital bed using either a computer coupled to the bed or using a graphical user interface of the bed. See U.S. Patent No. 7,154,397 in this regard. It has recently been contemplated to make a voice recording of a patient using a recorder included as part of a hospital bed or on other equipment at a point of care such that a patient verbally verifies their ID. See U.S. Provisional Application No. 61/752,100, filed January 14, 2013 and titled "Method and Apparatus for Collecting Patient Identification" (attorney docket no. 7175-221177). This same application contemplates using biometric data, such as a retinal scan or fingerprint, to verify the patient ID.

US2008120784A1 discloses a smart bed apparatus adapted to retain a patient wherein when a patient having a RFID device approaches a RFID antenna of the bed, the patient's identification information is sent from the RFID device of the patient to the RFID device of the bed in order to be sent to a bed computer and a server.

US2007120689A1 discloses a smart bed apparatus adapted to retain a patient wherein patient identification information is received from a RFID tag located on a patient and sent to a bed computer. wherein bed status conditions and patient data can be transmitted from bed to computer and made accessible through the hospital network.

US2012089419A1 discloses a hospital bed, communicating with an electronic medical record (EMR) system in healthcare facility.

WO2011100760A1 discloses a method of scanning a patient care area for tags for entities present in the area, a given patient being located in the area. The method may involve reading identity data from a tag, in response to detecting the tag for an entity in the area.

US2010174229A1 discloses A system and method for verifying identification of an authorized person by biometrics prior to a patient controlled analgesic medication delivery to the patient and for monitoring the delivery of medication to a patient.

WO2006035351A1 discloses a system for patient identification for association of wireless medical devices to patients.

As is apparent from the above discussion of the prior art, some current systems for associating a patient to a location (e.g., room) or to a piece of medical equipment (e.g., hospital bed) require some manner of extra action on the part of a caregiver or patient to make or verify the association. In some instances, redundant manual data entry is required by a caregiver and in other instances, additional information such as voice, finger print, or retina scan is required from a patient in order to make or verify the association. Thus, caregiver's would appreciate a healthcare communication system in which extra steps for patient association to a location and/or to equipment are eliminated.

An apparatus, system, or method, or a component or step thereof, may comprise one or more of the following features alone or in any combination.

According to one aspect of this disclosure, a system may include a bed that may have circuitry that, in turn, may include a reader. The system may have a wireless transmitter that may be configured to be worn by a patient and that may be configured to transmit a wireless signal that includes patient identification data. The system may further have an admission, discharge and transfer (ADT) computer and a bed status computer that may be in communication with the ADT computer and that may be in communication with the circuitry of the bed. The reader may be configured to receive the wireless signal from the wireless transmitter when the patient is within a threshold distance of the reader. The circuitry may automatically transmit the patient identification data included in the wireless signal to the bed status computer which may cooperate with the ADT system to automatically verify at least one of a patient-to-bed association and a patient-to-room association without the need for any manual data entry at the bed and without the need for any manual data entry at the bed status computer.

In some embodiments of the system, the circuitry of the bed may be configured to transmit bed data to the bed status computer for storage in memory of the bed status computer prior to the ADT computer making the verification. The system may further include an electronic medical records (EMR) computer and wherein the bed status computer may be configured to send the bed data to the EMR computer after the ADT computer makes the verification.

It is contemplated by this disclosure that the wireless transmitter may be included as part of a wristband worn by the patient. In such embodiments, the wrist band may include memory and the system may further include an encoder that may be operable to encode the patient identification information into the memory of the wristband for transmission by the wireless transmitter. The circuitry of the bed also may include a display screen and a name of the patient may be displayed on the display screen in response to the reader receiving the wireless signal. Furthermore, a verification message may be displayed on the display screen of the hospital bed after the ADT computer notifies the bed status computer of the verification and after the bed status computer notifies the circuitry of the bed of the verification.

According to another aspect of this disclosure, a bed may be provided for use in a healthcare facility that may have an admission, discharge and transfer (ADT) system in which a patient may be provided a wristband that may be configured to transmit a wireless identification signal that may include patient identification data. The healthcare facility may also have a bed status computer in communication with the ADT system. The bed may include a patient support structure that may support the patient. The bed may also have circuitry that may be coupled to the patient support structure. The bed may further have a receiver that may be coupled to the patient support structure and that may be coupled to the circuitry. The receiver may be configured to receive the wireless identification signal from the wristband when the patient is within a threshold distance of the receiver. The bed circuitry may automatically transmit the patient identification data included in the wireless identification signal to the bed status computer which may cooperate with the ADT system to automatically verify a patient-to-bed association without the need for any manual data entry at the bed and without the need for any manual data entry at the bed status computer.

In some embodiments, the bed may also have a user interface display screen that may be coupled to the patient support structure and that may be coupled to the circuitry. The user interface display screen may display a name of the patient in response to the receiver receiving the wireless identification signal from the wristband and the circuitry processing the identification signal. The circuitry may command the user interface display screen to display a verification message in response to the bed status computer sending a message to the circuitry indicating that the patient identification data has been verified.

According to this disclosure, the circuitry also may send bed data to the bed status computer. The bed data may include data relating to at least one patient physiological parameter, such as patient weight. The patient support structure may include a siderail and the user interface display screen may be coupled to the siderail. The receiver may be configured to receive radio frequency identification (RFID) signals.

According to another aspect of this disclosure, a method may include entering patient data manually at an admission, discharge and transfer (ADT) computer, encoding the patient data into memory of a wristband that may be worn by the patient, and receiving with a reader a wireless signal that may be transmitted by the wristband. The receiver may be located in a room that may be assigned to the patient. The method may further include displaying a name of the patient on a display screen of a hospital bed in response to the reader receiving the wireless signal, transmitting from circuitry of the bed to at least one bed status computer that may be remote from the bed at least some information that may be included in the wireless signal, communicating messages between the bed status computer and the ADT computer to verify that the patient is in the room that has been assigned. In this regard, verification may be achieved without the need for any manual data entry at the bed and without the need for any manual data entry at the bed status computer. The method may still further include communicating a verification message from the bed status computer to the circuitry of the bed and displaying a verification message on the display screen of the hospital bed.

The reader may be included as a component of the bed or it may be spaced from the bed. For example, in some embodiments, the reader may be included as a component of a locating and tracking system. It is contemplated that at least some of the information included in the wireless signal received by the reader may be sent to the bed status computer via a server of the locating and tracking system.

Also according to this disclosure, bed data may be sent to the bed status computer from the bed for storage in memory of the bed status computer prior to the ADT computer verifying that the patient is in the room that has been assigned. After the ADT computer verifies that the patient is in the room that has been assigned, the bed status computer may send the stored bed data to an electronic medical records (EMR) computer for storage in the patient's electronic medical record. Thereafter, the bed status computer may send or forward the bed data to the EMR computer on an as-received basis. The bed data may include data relating to bed parameters and/or data relating to patient parameters, such as patient physiological parameters.

The ADT computer may verify to the bed status computer that the patient is in the room that has been assigned without the need for any further manual data entry at the ADT computer. The patient data encoded into memory of the wristband may include, for example, a patient's name and a medical record number. Optionally, a room number of the assigned room may also be encoded into memory of the wristband.

The invention will now be further described by way of example with reference to the accompanying drawings, in which:
Fig. 1 is a diagrammatic view showing a process in which a patient is admitted at an ADT station having an ADT computer, a bed receives patient ID information transmitted wirelessly and sends it to bed status computer, the bed status computer automatically verifies the patient-to-room association with the ADT computer, and the bed status computer communicates the verification to the bed;

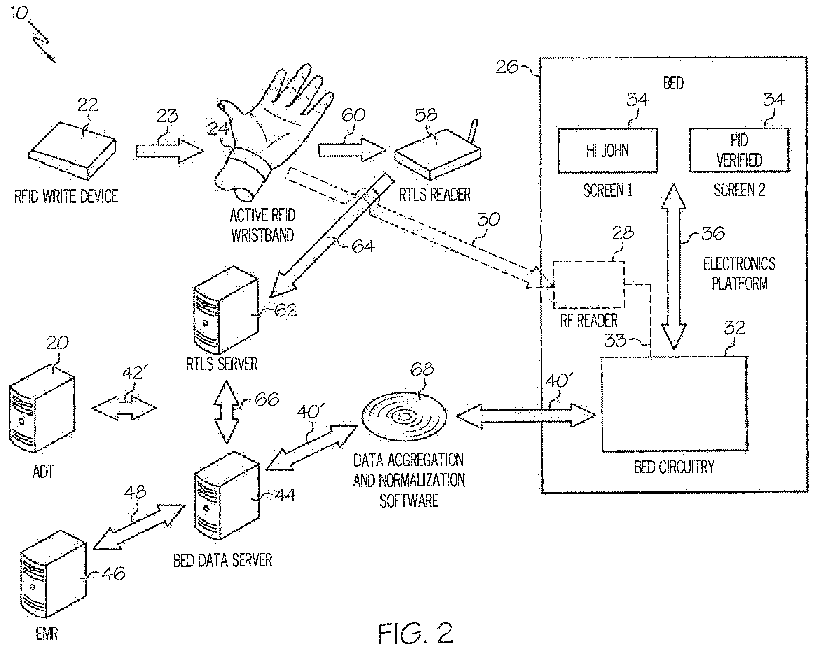

Fig. 2 is a diagrammatic view showing an RFID write device that operates to encode data, including patient ID data, onto a wristband worn by the patient, a real time locating system (RTLS) reader receiving a wireless transmission from the wristband and communicating data to an RTLS server which, in turn, communicates data to a bed data server, the bed data server sending data to the bed which displays the patient's name on a display screen of the bed, the bed data server also communicating with an ADT server to verify the patient-to-room association, and the bed data server communicating the verification to the bed which indicates the verification on the display screen;
Figs. 3A and 3B cooperate to form an activity diagram showing the activities and process steps of various people and various equipment of a direct patient ID association system in which a hospital bed has its own reader;
Figs. 4A and 4B cooperate to form an activity diagram showing the activities and process steps of various people and various equipment of a direct patient ID association system in which an RTLS reader in a room is used to sense patient presence in lieu of a reader included in a hospital bed; and
Figs. 5A and 5B cooperate to form an alternative activity diagram, similar to Figs. 3A and 3B, but with the bed having an Ethernet connection.

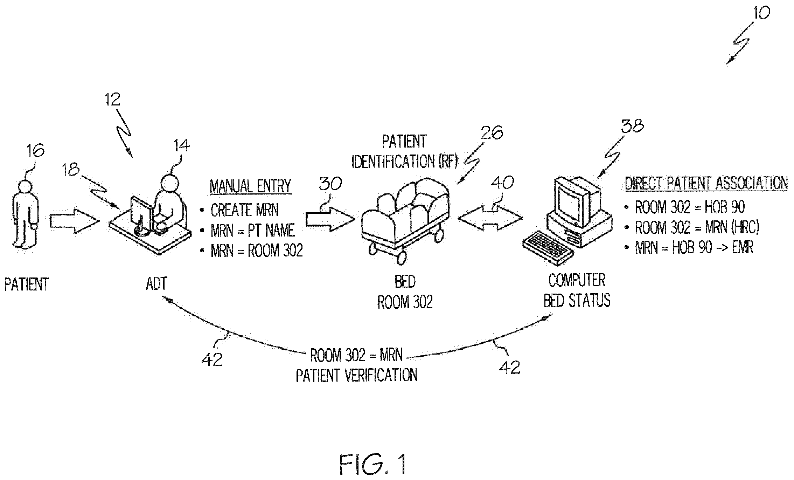

As shown in Figs. 1 and 2, a system 10 includes an ADT station 12 at which a caregiver 14 enters information about a patient 16 into an ADT computer 18 which is coupled to an ADT server 20. Using the ADT computer 18, the caregiver 14 creates or sets up a medical record number (MRN) for the patient 16. The caregiver 14 also manually enters the patient's name into the ADT computer 18 which links it with the MRN. Further, the caregiver 14 uses ADT computer 18 to assign a room to the patient such that the room assignment is linked with the MRN as indicated in Fig. 1. After the MRN, patient name, and room assignment are linked or associated in the ADT computer 18, the ADT computer 18 copies or transfers that information to a write device 22, shown in Fig. 2, which is then used to encode that information onto a wristband 24 to be worn by the patient 16.

In the illustrative example, radio frequency identification (RFID) technology is used by the write device 22 and the wristband 24 is an active RFID wristband that has memory to store the information sent wirelessly from write device 22 when the wristband 24 is in close proximity to the write device 22 as indicated diagrammatically by arrow 23 in Fig. 2. Transmission of data from write device 22 to memory of wristband 24 via a wired connection is also contemplated by this disclosure. Wristband 24 transmits wireless RFID signals that contain the information stored in the wristband 24. Thus, wristband 24 includes either a transceiver or a separate receiver and transmitter to receive and send wireless RFID signals.

According to a first embodiment of system 10, a hospital bed 26 includes a reader 28, such an RF reader, which receives a transmission of the wireless signal from wristband 24 when the wristband is within a threshold distance of the reader 28 as indicated diagrammatically in Fig. 2 with dotted arrow 30 (also indicated diagrammatically in Fig. 1 with solid arrow 30). The threshold distance depends upon a number of factors such as the frequency and power or signal strength of the wireless signal. However, a threshold distance on the order of about three to about five feet is contemplated as being suitable. Threshold distances greater or less than this range are within the scope of this disclosure. As stated previously, wristband 24 is "active" in the illustrative example in that it has a battery for powering the electrical components of the wristband, including the transceiver or transmitter. However, in other embodiments, a passive wristband may be used. In such embodiments, the passive wristband is placed much closer to the reader 28, such as on the order of a couple inches or less, because the passive wristband relies on energy from the reader 28 to reflect back data to the reader 28 as is known in the art.

As shown diagrammatically in Fig. 2, reader 28 is coupled to bed circuitry 32 as indicated by dotted line 33. Bed circuitry 32, in turn, is coupled to a display screen 34 as indicated diagrammatically by double headed arrow 36. Display screen 34 is a graphical user interface, such as a touch screen display, in some embodiments. Thus, screen 34 accepts user inputs for controlling features and functions of bed 26 as is known in the art. See, for example, U.S. Patent Application Publication Nos. 2012/0089419 A1 which is titled "Hospital Bed with Graphical User Interface Having Advanced Functionality" and 2008/0235872 A1 which is titled "User Interface for Hospital Bed,".

Bed circuitry 32 executes a software routine to process the data received by reader 28, including determining the name of the patient for display on display screen 34. Thus, in the illustrative example, display screen 34 has the message "Hi John" on screen 1 as shown in Fig. 2. In other embodiments, the patient's full name is shown. Of course, any suitable message such as "Welcome John" or "John Smith's Bed" or the like may be shown on display screen 34 within the scope of this disclosure.

In response to reader 28 reading the wireless signal from wristband 24, bed circuitry 32 of bed 26 transmits data, including bed ID data, the patient ID data and/or MRN, to a bed status computer 38 as indicated by double headed arrow 40 shown in Fig. 1. Bed status computer 38 is part of a nurse call system in some embodiments. For example, bed status computer 38 is a master nurse station computer in some embodiments. Bed status computer 38 then communicates with ADT computer 18, as indicated by arrows 42 in Fig. 1, to verify that the correct patient ID and/or MRN has been read by reader 28 of the bed 26 that is located at the room location assigned by the caregiver using ADT computer 18 initially. In some embodiments, such as the embodiment shown in Fig. 2, bed status computer 38 is communicatively coupled to a bed data server 44 which, in turn, is communicatively coupled to ADT server 20 via the infrastructure of the healthcare facility as indicated diagrammatically in Fig. 2 by double headed arrow 42'.

In the illustrative example, bed status computer 38 or bed data server 44 sends to the ADT computer 18 or the ADT server 20 the MRN sensed by reader 28 of bed 26 and the room number of the room in which bed 26 is located. ADT computer 18 compares the MRN and room number sent by bed status computer 38 with its records and if there is a match, a verification message is sent from the ADT computer 18 or server 20 to the bed status computer 38 or server 44 which, in turn, sends a message to bed circuitry 32 of bed 26 verifying that the correct patient is in bed 26 or, at least, near enough to bed 26 for the patient's wristband 24 to be sensed by reader 28.

In response to bed circuitry 32 receiving the verification message from computer 38 or server 44, the display screen 34 is changed to indicate that the patient ID has been verified as indicated by Screen 2 in Fig. 2. In the illustrative example, the words "PID Verified" are shown on display screen 34, with PID being an acronym for "patient ID." However, any suitable message may be displayed on display screen 34 to indicate the verification such as "Bed Assignment Verified for John" or "This is the correct bed for John Smith" just to mention a couple possibilities. Based on the foregoing, it should be appreciated that, in Fig. 2, the Screen 1 and Screen 2 messages are displayed on the same display screen 34, but at different times. The message of Screen 1 is shown first in response to reader 28 first receiving the wireless signal with data from the patient's wrist band 24 and then, the message of Screen 2 is shown later after computers 18, 38 and/or servers 20, 44 of system 10 have cooperated to verify that the patient is on or near the correct bed 26 corresponding to the assigned room location.

It should be understood that, typically, ADT computer 18 and bed status computer 38 are each located remotely from bed 26 and from each other. The corresponding servers 20, 44 are located remotely from bed 26 as well. Thus, communications infrastructure is, of course, present in the healthcare facility to communicatively couple or link together bed 26, computers 18, 38, and/or servers 20, 44. Such infrastructure typically includes, for example, wires, cables, jacks, routers, gateways, switches, etc. as well as wireless communication components such as wireless access points. In some embodiments, such as the embodiment shown in Fig. 2, bed status computer 38 is communicatively coupled to bed data server 44 which, in turn, is communicatively coupled to ADT server 20 via the infrastructure of the healthcare facility. The communication infrastructure of the healthcare facility includes infrastructure operating as for example, an Ethernet, a wide area network (WAN), a local area (LAN) and the like. Thus, any manner of communicatively interconnecting bed 26 with computers 18, 38 and/or servers 20, 44 is intended to be within the scope of this disclosure.

According to some embodiments of this disclosure, bed 26 couples to a network interface unit (NIU) of a wireless communication module (WCM) which serves as a location unit or module. WCM's are sometimes referred to as wireless interface units (WIU's). Details of such devices are shown and described in U.S. Patent Nos. 7,852,208 and 7,319,386. In embodiments having NIU's, a detachable cable extends between a cable connection port on bed 26 and a cable connection port of the NIU. The NIU is mounted to a wall in the room or to some other architectural structure such as, for example, a headwall unit, column, or bed locator unit that are intended to remain in the room on an ongoing basis.

The NIU has an NIU ID, which serves as a location ID, that is sent to computer 38 along with the data received from bed 26. The location ID from the NIU corresponds to the room location. It is not uncommon for two or more patient beds to be located in the same room in a healthcare facility. By providing such rooms with two or more NIU's that couple to respective beds 26, it is possible for computers 18, 38 to keep track of which bed 26 (e.g., an A bed or a B bed) is sending data for which patient because of the unique location ID's sent by the respective NIU's along with the data. Furthermore, the specific bed 26 (e.g., an A bed or B bed) within the room can be assigned at the ADT computer 18 to a patient. If the patient goes to the correct room, but moves near or gets into the wrong bed 26 which reads the patient's wristband, a non-verification message is sent by ADT computer 18 to bed status computer 38 and then on to bed 26 for display on screen 34. In response to the non-verification message appearing on display screen 34, the patient or caregiver can then take corrective action to move the patient to the proper, assigned bed 26.

It is contemplated by this disclosure that after reader 28 of bed 26 reads the wireless signal from a patient's wristband 24 and, optionally, after bed 26 senses that the patient is in the bed such as via a weigh scale system of the bed sensing weight in excess of a threshold amount, bed 26 periodically sends bed data to bed status computer 38 and/or server 44. Computer 38 or server 44 stores the bed data periodically received from bed 26 until ADT computer or server 20 sends a message verifying the patient ID for the particular bed 26. In response to the verification message being received for the particular patient, bed status computer 38 and/or server 44 transmits the accumulated bed data for the particular patient to an electronic medical records (EMR) computer 46 for storage in the patient's EMR as indicated diagrammatically in Fig. 2 via double headed arrow 48. Thus, if there is a time delay between the time at which a patient arrives at the assigned bed 26 and the time at the patient ID is verified, the bed data generated during that time delay is not lost. The bed data may include information concerning bed parameters or bed status (e.g., up/down positions of siderails of the bed, operational status of a bed exit or patient position monitor alarm, braked/released status of casters of the bed, operational status of a therapy surface of the bed, etc.) and/or information concerning patient parameters (e.g., patient weight, heart rate, blood pressure, blood oxygenation level, respiration rate, etc.).

In some embodiments of system 10, the wireless transmissions from wristband 24 are not read by reader 28 of bed 26 but instead, are read by a reader 58 of a real time locating system (RTLS) as indicated diagrammatically in Fig. 2 by arrow 60. In such embodiments, therefore, beds 26 are not required to have any reader 28. The data from wristband 24 that is read by reader 58 is, in turn, transmitted to an RTLS server 62 as indicated diagrammatically in Fig. 2 by arrow 64. Server 62 then passes the information from wristband 24 on to bed data server 44 as indicated diagrammatically in Fig. 2 by arrow 66. Bed data server 44 then communicates some or all of the data from wristband 24 to bed circuitry 32 of the bed 26 that is located in the same room as RTLS reader 58. This is indicated diagrammatically in Fig. 2 by double headed arrows 40'. Thus, reader 58 is in the same room as the bed 26 to which the patient having wristband 24 was assigned, whereas servers 44, 62 are located remotely from the assigned room. After bed circuitry 34 receives the patient ID or name information from server 44, circuitry 34 then commands display screen 34 to display the patient's name and to automatically take the steps to verify the patient ID or name or MRN with the ADT computer 18 or server 20 in the same manner as discussed above.

In Fig. 2, data aggregation and normalization software 68 is shown diagrammatically between arrows 40'. This software is used to convert formatting of data sent to circuitry 32 of bed 26 as well as data sent from circuitry 32 of bed 26, from one format to another. For example, a healthcare facility oftentimes includes beds 26 that are different makes and models, and even beds 26 from different bed manufacturers. The data formatting for each of these various types of beds is not consistent. Some beds have features that are omitted from other beds and thus, the data streams will be custom designed for each type of bed. Accordingly, the patient ID, name, MRN, and any other data encoded on wristband 24 may need to be formatted differently depending upon the type of bed 26 to which it is being sent by server 44. For example, the data may be required by the particular bed to be located at particular frames within a transmitted data packet or packets in order for circuitry 32 to receive and process the data properly. Software 68 handles that formatting. Similarly, server 44 may have its own formatting for receiving data from beds 26.

Software 68 is included on server 44 in some embodiments and is included on another server (not shown) in other embodiments. Additional details concerning software 68 is found in U.S. Patent Application Publication No. 2012/0316892 A1 which it titled "System and Method of Bed Data Aggregation, Normalization and Communication to Third Parties". One example of software 68 is the NAVICARE® SMARTSYNC™ software marketed by Hill-Rom Company Inc. which converts bed data into the health level 7 (HL7) format which is suitable for transmission to hospital EMR systems of which server 46 is a part according to this disclosure.

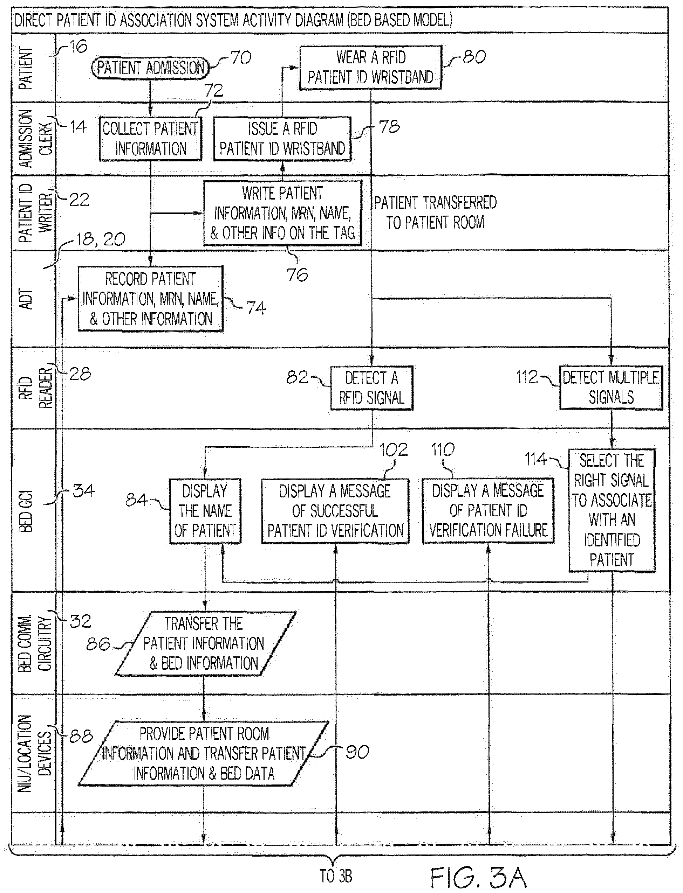

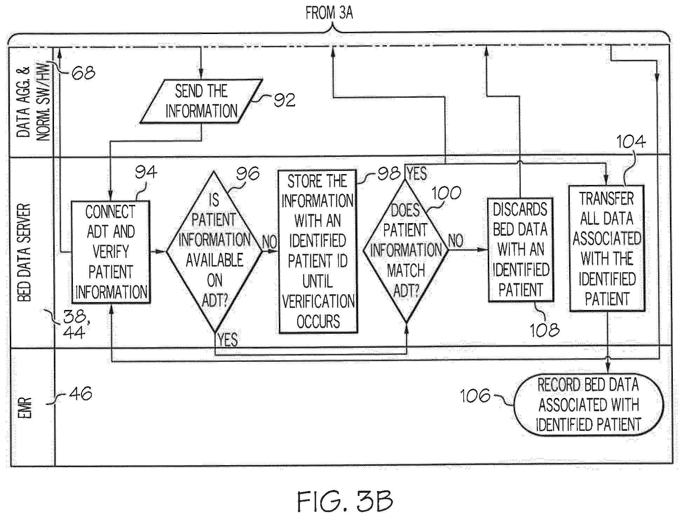

Referring now to Figs. 3A and 3B, an activity diagram shows the various activities and process steps of various people and various equipment of direct patient ID association system 10 in which hospital bed 26 has its own reader 28. Thus, Figs. 3A and 3B represent the steps associated with system 10 that were discussed above in connection with the embodiment in which bed 26 includes reader 28, but are presented in a different manner to enhance the understanding of system 10. As indicated at block 70 a patient 16 goes to a patient admission area and an admission clerk 14 collects patient information as indicated at block 72. As clerk 14 enters the patient's information, ADT computer 18 and/or server 20 records the patient information including the patient's name and MRN as indicated at block 74.

The clerk 14 also uses writer 22 to encode or write the patient information including MRN, name, and other information onto a tag of a wristband as indicated at block 76 of Fig. 3A. The clerk 14 then issues the RFID Patient ID Wristband 24 to the patient 16 as indicated at block 78. The patient 16 wears the wristband 24 as indicated at block 80. The patient is then transferred to the patient room at which reader 28 of bed 26 detects an RFID signal from wristband 24 as indicated at block 82.

After reader 34 detects the signal from wristband 24 at block 82, the graphical caregiver interface (GCI), which is another term referring to display 34 of bed 26, displays the name of the patient as indicated at block 84 and the bed communication circuitry, which is included as part of bed circuitry 32, transfers patient information received from wristband 24 and bed information from bed 26 as indicated at block 86. The patient information and bed information passes through an NIU or location device 88 which provides patient room information (e.g., an ID of device 88) along with the patient information and bed information, as indicated at block 90 in Fig. 3A, to whatever server (e.g., hardware) of system 10 has data aggregation and normalization software 68 which, in turn, sends the information after formatting conversion, if necessary, to bed data computer or server 38, 44 as indicated at block 92 of Fig. 3B.

After receiving the patient information, bed information, and location information, computer 38 and/or server 44 connects to the ADT computer 18 or server 20 and verifies whether the patient information is correct as indicated at block 94 of Fig. 3B. As indicated at block 96, bed data computer 38 or server 44 determines whether the patient information is available from ADT computer 18 or server 20. If the patient information is not yet available from ADT computer 18 or server 20, bed data computer 38 or server 44 stores the information and associates the information with the identified patient until verification occurs as indicated at block 98.

If at block 96, computer 38 or server 44 determines that patient information is available on ADT computer 18 or server 20, then computer 38 or server 44 determines whether the patient information received from bed 26 matches the patient information on ADT computer 18 or server 20 as indicated at block 100. If the patient data matches, then bed status computer 38 or server 44 communicates a message back to circuitry 32 of bed 26 which results in GCI 34 displaying a message of successful patient ID verification as indicated at block 102 in Fig. 3A. Also, if the patient data matches at block 100, computer 38 or server 44 transfers all data associated with the identified patient to the EMR server 46 as indicated at block 104 and then, EMR server 46 stores the bed data associated with the identified patient as indicated at block 106. In a variant embodiment, some but not all of the data is transferred to the EMR server 46 at block 104 rather than all of the data. For example, there may be some bed data in which the EMR system has no interest or capability of storing.

If at block 100, the patient information from bed 26 does not match the patient information of the ADT system, then bed status computer 38 or server 44 discards the bed data associated with the non-matching patient as indicated at block 108 in Fig. 3B and communicates a message back to circuitry 32 of bed 26 which results in GCI 34 displaying a message of a failure to verify the patient ID as indicated at block 110 in Fig. 3A. Figs. 3A and 3B also show steps that occur in the rare instance when reader 28 concurrently detects multiple signals from multiple patients wearing wristbands 24 as indicated at block 112 in Fig. 3A. Under that scenario, GCI 34 of bed 26 displays a message indicating the multiple patient names and instructing a user, such as a caregiver or one of the patients, to select the right name of the patient whose patient information should be associated with the bed 26 as indicated at block 114. Once the selection of the patient's name is made at block 114, circuitry 32 of bed 26 communicates the selection to bed data computer 38 or server 44 which then proceeds with the step of block 94 and display 34 proceeds to step 84 to display the patient's name. In an alternative embodiment, bed 26 sends all of the patient ID's sensed by reader 28 to bed data computer 38 or server 40 which then communicates with ADT computer 18 or server 20 to determine which of the multitude of patient ID's is the one that has been assigned to bed 26 and then circuitry 32 commands GCI 34 to display a message indicating which patient is the patient that is assigned to bed 26. Of course, if none of the patient ID's match, then an appropriate message indicating a validation failure is displayed on GCI 34.

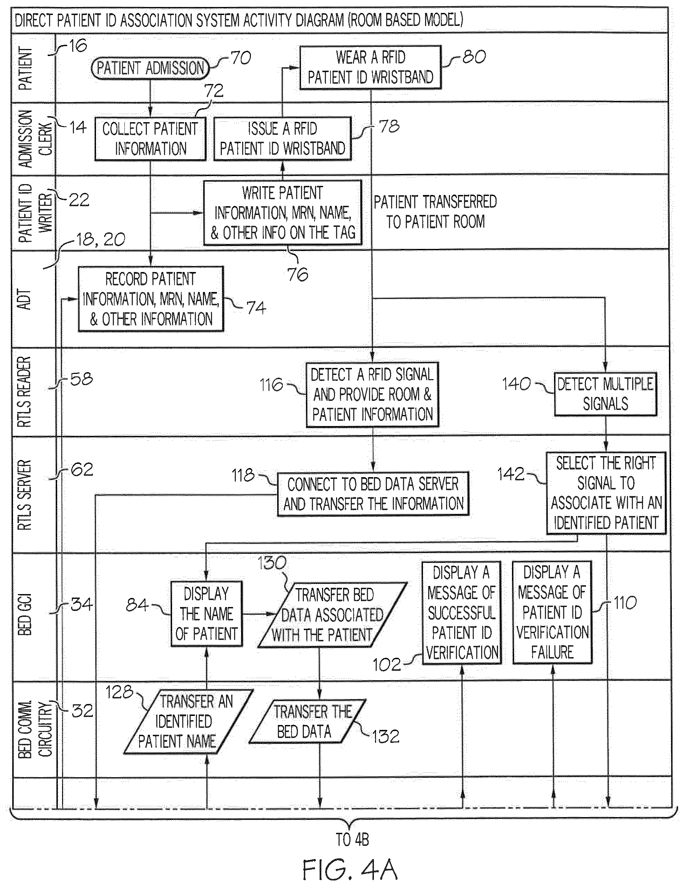

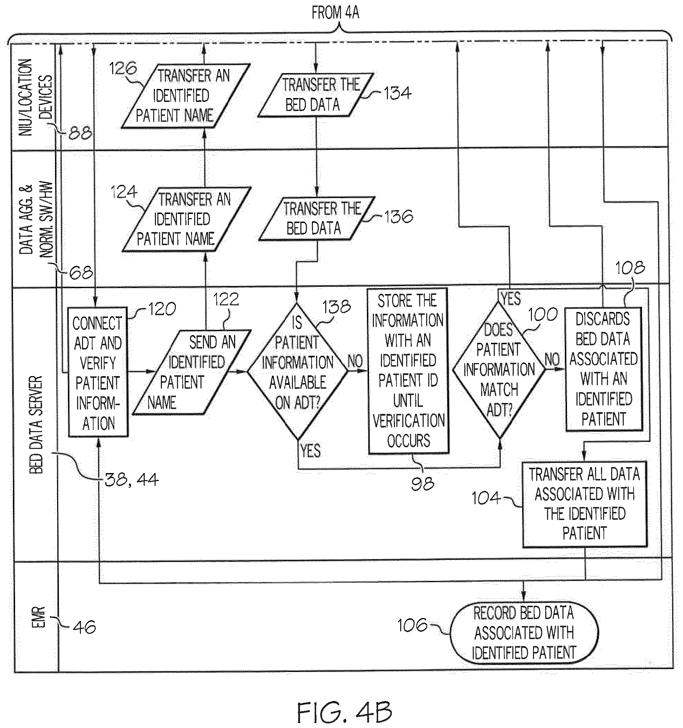

Referring now to Figs. 4A and 4B, an activity diagram showing the activities and process steps of various people and various equipment of direct patient ID association system 10 in which an RTLS reader 58 in a room is used to sense patient presence in lieu of reader 28 included in hospital bed 26. In Figs. 4A and 4B, several of the individuals, steps, and/or equipment involved is identical to that shown in Figs. 3A and 3B. In such instances, like reference numerals are used to denote the like individuals, steps, and/or equipment and a description of these is either omitted or is abbreviated. However, the above description of these like aspects is equally applicable. Thus, the following discussion focuses on the differences between Figs. 3A and 3B and Figs. 4A and 4B.

When RTLS reader 58 detects an RFID signal from wristband 24 it sends room information (e.g., an ID of reader 58) and the patient information from wristband 24 to RTLS server 62 as indicated at block 116 of Fig. 4A. Server 62 then communicates with bed data computer 38 or server 44 and transfers the room information and patient information as indicated at block 118. Computer 38 or server 44 then communicates with ADT computer 18 or server 20 to verify the patient information as indicated as block 120 in Fig. 4B. As indicated at block 122, bed data computer 38 or server 44 also sends the identified patient name to whatever server (e.g., hardware) of system 10 has data aggregation and normalization software 68 which, in turn, sends the patient name after formatting conversion, if necessary, to NIU or location device 88 as indicated at block 124 of Fig. 4B. Device 88 transfers the identified patient name to bed circuitry 32 as indicated at block 126 of Fig. 4B. Circuitry 32 then transfers the identified patient name to the display 34 as indicated at block 128 in Fig. 4A which, in turn, displays the name of the patient as indicated at block 84.

The bed GCI or display 34 and bed circuitry 32 cooperate to transfer bed data, as indicated at blocks 130, 132, respectively, in Fig. 4A to the NIU or location device 88. Location device 88, then transfers the bed data to the data aggregation and normalization software and hardware 68 as indicated at block 134 of Fig. 4B. Software/hardware 68 then transfers the bed data to bed computer 38 or server 44 as indicated at block 136. As shown at block 138 in Fig. 4B, computer 38 or server 44 checks to see if the patient information is available on ADT computer 18 or server 20 and then proceeds through blocks 98, 100, 102, 104, 106, 108 and 110 of Figs. 4A and 4B in the same manner as described above with regard to Figs. 3A and 3B.

In the method of Figs. 4A and 4B, if reader 58 detects multiple signals from wristbands 24 of multiple patients, as indicated at block 140 in Fig. 4A, RTLS server 62 is used to select the right signal to associate with an identified patient as indicated at block 142 of Fig. 4A. The selection can either be made manually by a caregiver using a keyboard and display screen, for example, associated with server 62. Alternatively or additionally, server 62 includes software in some embodiments that make the selection. Such software analyzes signal strength from wristbands 24 in some embodiments. Alternatively or additionally, server 62 selects the patient that is closest to the bed 26 as determined by signal strength, time of flight, time of arrival, or other parameters of the signal from wristbands 24. Once the selection is made by server 62, the method of Figs. 4A and 4B proceeds to block 120 and the subsequent blocks thereafter.

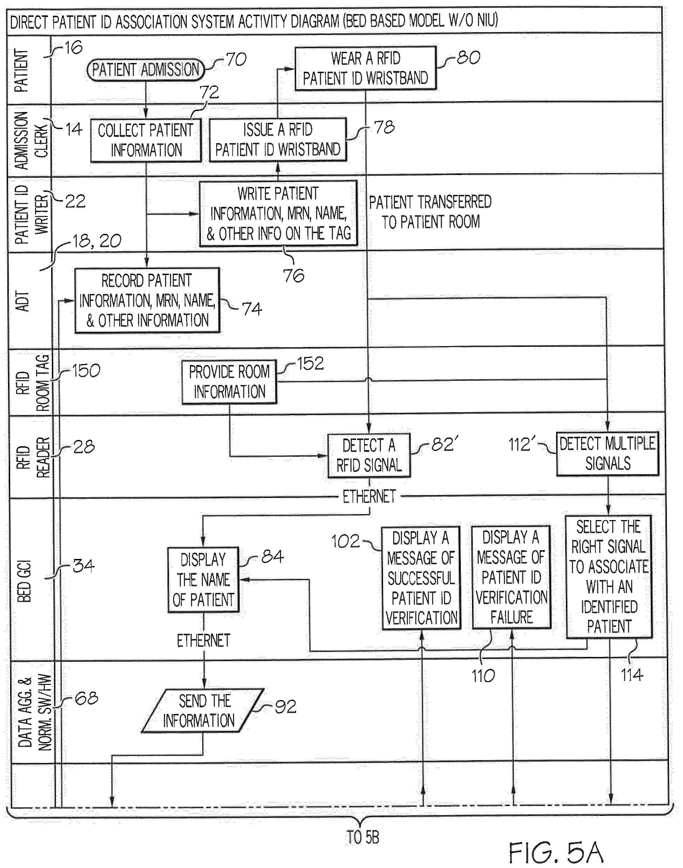

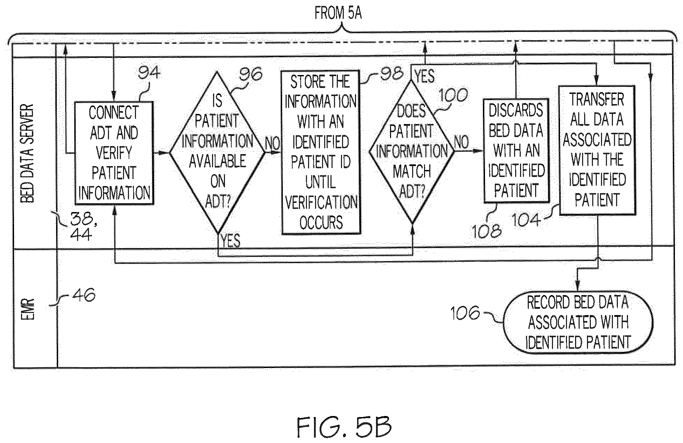

Referring now to Figs. 5A and 5B, an activity diagram showing the activities and process steps of various people and various equipment of direct patient ID association system 10 in which NIU/location device 88 is omitted and bed 26 connects directly to the Ethernet of a healthcare facility. In some embodiments, such connections to the Ethernet are accomplished with bidirectional wireless communications between circuitry 32 of bed 26 and one or more wireless access points within reception range of the bed 26. Alternatively or additionally, circuitry 32 may connect to the Ethernet with a wired connection from bed 26 to an Ethernet jack or port. In Figs. 5A and 5B, several of the individuals, steps, and/or equipment involved is identical to that shown in Figs. 3A and 3B. In such instances, like reference numerals are used to denote the like individuals, steps, and/or equipment and a description of these is either omitted or is abbreviated. However, the above description of these like aspects is equally applicable. Thus, the following discussion focuses on the differences between Figs. 3A and 3B and Figs. 5A and 5B.

An RFID tag 150, such as one mounted at a generally fixed location in a patient room, provides room information (e.g., a tag ID) wirelessly to RFID reader 28 of bed 26 as indicated at block 152 in Fig. 5A. Thus, reader 28 detects an RFID signal from wristband 24 of the patient 16 and from RFID tag 150 as indicated at block 82' of Fig. 5A. An Ethernet protocol is then used to transfer the patient information and room information to bed GCI 34 which displays the name of the patient as indicated at block 84. An Ethernet connection is then used to transfer the information from bed GCI to software/hardware 68 which proceeds to block 92 and the subsequent blocks in the same manner as described above in connection with Figs. 3A and 3B, although bed data computer 38 or computer 44 communicates via an Ethernet connection with bed GCI 34.

As shown in Fig. 5A at block 112', a scenario is possible in which RFID reader 28 of bed 26 receives RFID signals from multiple wristbands 24 and from multiple RFID room tags 150. For example, one room tag 150 may be associated with an A portion of a room and another room tag 150 may be associated with a B portion of the room. Under that scenario, GCI 34 of bed 26 displays a message indicating the multiple patient names and multiple room locations and instructing a user, such as a caregiver or one of the patients, to select the right room location (e.g., room 302A or 302B) and the right name of the patient whose patient information should be associated with the bed 26 as indicated at block 114. After block 114, the method proceeds to block 94 and proceed from there. In an alternative embodiment, bed 26 sends all of the patient ID's and room ID's sensed by reader 28 to bed data computer 38 or server 40 which then communicates with ADT computer 18 or server 20 to determine which of the multitude of patient ID's is the one that has been assigned to bed 26 and then GCI 34 is commanded to display a message indicating which patient is the patient that is assigned to bed 26 at the proper room location. Of course, if none of the patient ID's match, then an appropriate message indicating a validation failure is displayed on GCI 34.

Although certain illustrative embodiments have been described in detail above, variations and modifications exist.

## Claims

1. A system (10) comprising
a bed (26) having circuitry (32) including a reader (28),
a wireless transmitter configured to be worn by a patient (16) and configured to transmit a wireless signal that includes patient identification data,
an admission, discharge and transfer (ADT) computer (18, 20), and
a bed status computer (38) in communication with the ADT computer and in communication with the circuitry of the bed, wherein the reader is configured to receive the wireless signal from the wireless transmitter when the patient is within a threshold distance of the reader, wherein the circuitry automatically transmits the patient identification data included in the wireless signal to the bed status computer which cooperates with the ADT computer to automatically verify at least one of a patient-to-bed association and a patient-to-room association without the need for any manual data entry at the bed and without the need for any manual data entry at the bed status computer, wherein the circuitry of the bed is configured to transmit bed data to the bed status computer for storage in memory of the bed status computer prior to the ADT computer making the verification, wherein the system further comprises an electronic medical records (EMR) computer (46), and wherein the bed status computer is configured to send the bed data to the EMR computer after the ADT computer makes the verification.

2. The system (10) of claim 1, wherein the wireless transmitter is included as part of a wristband (24) worn by the patient (16).

3. The system (10) of claim 2, wherein the wrist band (24) includes memory and further comprising an encoder operable to encode the patient identification information into the memory of the wristband for transmission by the wireless transmitter.

4. The system (10) of any preceding claim, wherein the circuitry (32) of the bed (26) also includes a display screen (34) and a name of the patient (16) is displayed on the display screen in response to the reader (28) receiving the wireless signal.

5. The system (10) of claim 4, wherein a verification message is displayed on the display screen (34) of the bed (26) after the ADT computer (18, 20) notifies the bed status computer (38) of the verification and after the bed status computer notifies the circuitry(32) of the bed of the verification.

6. The system (10) of claim 1, further comprising a user interface display screen (34) coupled to the circuitry (32), wherein the user interface display screen displays a name of the patient (16) in response to the circuitry processing the wireless signal.

7. The system (10) of claim 6, wherein the circuitry (32) commands the user interface display screen (34) to display a verification message in response to the bed status computer (38) sending a message to the circuitry indicating that the patient identification data has been verified.

8. The system (10) of claim 7, wherein the bed data includes data relating to at least one patient physiological parameter.

9. The system (10) of claim 8, wherein the at least one patient physiological parameter includes patient weight.

10. The system(10) of any one of claims 6 to 9, wherein the bed (26) includes a siderail and the user interface display screen (34) is coupled to the siderail.

11. The system (10) of any preceding claim, wherein the reader (28) is configured to receive radio frequency identification (RFID) signals.

## Patentansprüche

1. Ein System (10), umfassend
ein Bett (26) mit einem Schaltkreis (32) einschließlich eines Lesegeräts (28),
ein drahtloses Übermittlungsgerät, dass so konfiguriert ist, dass es von einem Patienten (16) getragen wird, und so konfiguriert ist, dass es ein drahtloses Signal übermittelt, das Patientenidentifikationsdaten beinhaltet,
einen Computer (18, 20) für die Aufnahme, Entlassung und Verlegung (Admission, Discharge und Transfer; ADT), und
einen Computer für den Bettstatus (38) in Kommunikation mit dem ADT-Computer und in Kommunikation mit dem Schaltkreis des Bettes, wobei das Lesegerät so konfiguriert ist, dass es ein drahtloses Signal vom drahtlosen Übermittlungsgerät empfängt, wenn sich der Patient innerhalb eines Grenzwerts für den Abstand des Lesegeräts befindet, wobei der Schaltkreis die Patientenidentifikationsdaten, die das drahtlose Signal beinhaltet, automatisch an den Computer für den Bettstatus übermittelt, der mit dem ADT-Computer zusammenarbeitet, um automatisch mindestens eine Patient-zu-Bett-Zuordnung und eine Patient-zu-Raum-Zuordnung zu verifizieren, ohne dass am Bett manuell Daten eingegeben werden müssen und ohne dass am Computer für den Bettstatus manuell Daten eingegeben werden müssen, wobei der Schaltkreis des Bettes so konfiguriert ist, dass er Bettdaten an den Computer für den Bettstatus zur Speicherung im Speicher des Computers für den Bettstatus übermitteln kann, bevor der ADT-Computer die Verifizierung vornimmt, wobei das System weiter einen Computer für elektronische Patientenakten (EPA) (46) umfasst, und wobei der Computer für den Bettstatus so konfiguriert ist, dass er Bettdaten an den EPA-Computer sendet, nachdem der ADT-Computer die Verifizierung vorgenommen hat.

2. Das System (10) nach Anspruch 1, wobei sich das drahtlose Übermittlungsgerät als Teil in einem Armband (24) befindet, das von dem Patienten (16) getragen wird.

3. Das System (10) nach Anspruch 2, wobei das Armband (24) einen Speicher beinhaltet und weiter ein Kodiergerät umfasst, das einsatzfähig ist, um die Patientenidentifikationsinformation in den Speicher des Armbands für die Übermittlung durch das drahtlose Übermittlungsgerät zu kodieren.

4. Das System (10) nach einem der vorstehenden Ansprüche, wobei der Schaltkreis (32) des Bettes (26) auch eine Bildschirmanzeige (34) einschließt und der Name eines Patienten (16) auf der Bildschirmanzeige als Reaktion auf das Lesegerät (28), das ein drahtloses Signal empfängt, angezeigt wird.

5. Das System (10) nach Anspruch 4, wobei eine Verifizierungsmeldung auf der Bildschirmanzeige (34) des Bettes (26) angezeigt wird, nachdem der ADT-Computer (18, 20) dem Computer für den Bettstatus (38) die Verifizierung meldet und nachdem der Computer für den Bettstatus dem Schaltkreis (32) des Bettes die Verifizierung meldet.

6. Das System (10) nach Anspruch 1, weiter umfassend eine Bildschirmanzeige der Benutzeroberfläche (34), die an den Schaltkreis (32) gekoppelt ist, wobei die Bildschirmanzeige der Benutzeroberfläche den Namen eines Patienten (16) als Reaktion auf den Schaltkreis, der das drahtlose Signals verarbeitet, anzeigt.

7. Das System (10) nach Anspruch 6, wobei der Schaltkreis (32) der Bildschirmanzeige der Benutzeroberfläche (34) befiehlt, eine Verifizierungsmeldung als Reaktion auf den Computer für den Bettstatus (38) anzuzeigen, der eine Meldung an den Schaltkreis sendet, die anzeigt, dass die Patientenidentifikationsdaten verifiziert wurden.

8. Das System (10) nach Anspruch 7, wobei die Bettdaten Daten beinhalten, die mindestens einen physiologischen Parameter eines Patienten betreffen.

9. Das System (10) nach Anspruch 8, wobei der mindestens eine physiologische Parameter eines Patienten das Patientengewicht beinhaltet.

10. Das System (10) nach einem der Ansprüche 6 bis 9, wobei das Bett (26) eine Seitensicherung beinhaltet und die Bildschirmanzeige der Benutzeroberfläche (34) an die Seitensicherung gekoppelt ist.

11. Das System (10) nach einem der vorstehenden Ansprüche, wobei das Lesegerät so konfiguriert ist, dass es Signale zur Radiofrequenz-Identifikation (RFID) empfangen kann.

## Revendications

1. Système (10) comprenant
un lit (26) ayant un ensemble de circuits (32) incluant un lecteur (28),
un émetteur sans fil conçu pour être porté par un patient (16) et conçu pour émettre un signal sans fil qui inclut des données d'identification du patient,
un ordinateur d'admission, de décharge et de transfert (ADT) (18, 20), et
un ordinateur d'état de lit (38) en communication avec l'ordinateur ADT et en communication avec l'ensemble de circuits du lit, dans lequel le lecteur est conçu pour recevoir le signal sans fil provenant de l'émetteur sans fil lorsque le patient est dans un rayon inférieur à une distance seuil du lecteur, dans lequel l'ensemble de circuits transmet automatiquement les données d'identification du patient incluses dans le signal sans fil à l'ordinateur d'état de lit qui coopère avec l'ordinateur ADT pour vérifier automatiquement au moins une d'une association patient-lit et d'une association patient-chambre sans avoir besoin d'aucune entrée manuelle de données au niveau du lit et sans avoir besoin d'aucune entrée manuelle de données sur l'ordinateur d'état de lit, dans lequel l'ensemble de circuits du lit est conçu pour transmettre des données de lit à l'ordinateur d'état de lit pour qu'elles soient enregistrées dans la mémoire de l'ordinateur d'état de lit avant que l'ordinateur ADT fasse la vérification, dans lequel le système comprend en outre un ordinateur de dossiers médicaux électroniques (DME) (46), et dans lequel l'ordinateur d'état de lit est conçu pour envoyer les données de lit à l'ordinateur EMR après que l'ordinateur ADT a fait la vérification.

2. Système (10) selon la revendication 1, dans lequel l'émetteur sans fil est inclus en tant que partie d'un bracelet (24) porté par le patient (16).

3. Système (10) selon la revendication 2, dans lequel le bracelet (24) inclut la mémoire et comprend en outre un encodeur pouvant être utilisé pour encoder les informations d'identification du patient dans la mémoire du bracelet en vue de leur émission par l'émetteur sans fil.

4. Système (10) selon l'une quelconque des revendications précédentes, dans lequel l'ensemble de circuits (32) du lit (26) inclut également un écran d'affichage (34), le nom du patient (16) s'affichant sur l'écran d'affichage en réponse à la réception du signal sans fil par le lecteur (28).

5. Système (10) selon la revendication 4, dans lequel un message de vérification s'affiche sur l'écran d'affichage (34) du lit (26) après que l'ordinateur ADT (18, 20) informe l'ordinateur d'état de lit (38) de la vérification et après que l'ordinateur d'état de lit informe l'ensemble de circuits (32) du lit de la vérification.

6. Système (10) selon la revendication 1, comprenant en outre un écran d'affichage d'interface utilisateur (34) couplé à l'ensemble de circuits (32), dans lequel l'écran d'affichage d'interface utilisateur affiche le nom du patient (16) en réponse au traitement du signal sans fil par l'ensemble de circuits.

7. Système (10) selon la revendication 6, dans lequel l'ensemble de circuits (32) commande l'écran d'affichage d'interface utilisateur (34) pour qu'il affiche un message de vérification en réponse à l'envoi, par l'ordinateur d'état de lit (38), à l'ensemble de circuits d'un message indiquant que les données d'identification du patient ont été vérifiées.

8. Système (10) selon la revendication 7, dans lequel les données de lit incluent des données relatives à au moins un paramètre physiologique du patient.

9. Système (10) selon la revendication 8, dans lequel le ou les paramètres physiologiques du patient incluent le poids du patient.

10. Système (10) selon l'une quelconque des revendications 6 à 9, dans lequel le lit (26) inclut une ridelle et l'écran d'affichage d'interface utilisateur (34) est couplé à la ridelle.

11. Système (10) selon l'une quelconque des revendications précédentes, dans lequel le lecteur est conçu pour recevoir des signaux d'identification par radiofréquence (RFID).
